# NEW EUROPEAN PATENT SPECIFICATION

(11) **EP 1 702 641 B2**
(45) Date of publication and mention of the opposition decision: **05.08.2020**
(45) Mention of the grant of the patent: 11.08.2010
(21) Application number: 06251331.2
(22) Date of filing: 14.03.2006
(51) Int. Cl.: A61B 5/03, A61B 5/07, A61M 27/00

(54) **Pressure sensing devices**
Druckmessvorrichtung
Dispositif de mesure de pression

(30) Priority: 15.03.2005 US 661758 P; 11.04.2005 US 907663; 11.04.2005 US 907664; 11.04.2005 US 907665
(43) Date of publication of application: 20.09.2006
(73) Proprietor: Codman & Shurtleff, Inc., Raynham, Massachusetts 02767 (US)
(72) Inventor: Mauge, Christophe, Melrose Massachusetts 02176 (US); Dextradeur, Alan J., Franklin Massachusetts 02038 (US); McCusker, Daniel J., Bridgewater Massachusetts 02324 (US); Meyer, Stefan, 35236 Breidenbach (DE); Boedecker, Volker, 30177 Hannover (DE); Ostermeier, Max G., 30177 Hannover (DE); Kraus, Robert G., Attleboro Massachusetts 02703 (US)
(74) Representative: Tunstall, Christopher Stephen

(56) References cited:
- EP-A- 0 115 548
- EP-A- 1 389 477
- EP-A2- 1 512 427
- WO-A-91/05575
- WO-A2-02/062215
- DE-A1- 10 239 743
- US-A- 4 718 425
- US-A- 4 850 358
- US-A- 5 704 352
- US-A- 5 957 912
- US-A- 6 120 457
- US-A1- 2002 035 331
- US-A1- 2002 087 059
- US-A1- 2003 135 110
- US-A1- 2004 193 021
- US-B1- 6 264 611
- US-B2- 10 156 469

## Description

The present invention relates to ventricular catheters, and in particular to a catheter device having a pressure sensor disposed therein. A pressure sensing ventricular catheter is disclosed for example in EP-A-1 512 427.

Hydrocephalus is a neurological condition that is caused by the abnormal accumulation of cerebrospinal fluid (CSF) within the ventricles, or cavities, of the brain. CSF is a clear, colorless fluid that is primarily produced by the choroid plexus and surrounds the brain and spinal cord. CSF constantly circulates through the ventricular system of the brain and is ultimately absorbed into the bloodstream. CSF aids in the protection of the brain and spinal cord. Because CSF keeps the brain and spinal cord buoyant, it acts as a protective cushion or "shock absorber" to prevent injuries to the central nervous system.

Hydrocephalus, which affects children and adults, arises when the normal drainage of CSF in the brain is blocked in some way. Such blockage can be caused by a number of factors, including, for example, genetic predisposition, intra-ventricular or intra-cranial hemorrhage, infections such as meningitis, head trauma, or the like. Blockage of the flow of CSF consequently creates an imbalance between the amount of CSF produced by the choroid plexus and the rate at which CSF is absorbed into the bloodstream, thereby increasing pressure on the brain, which causes the ventricles to enlarge.

Hydrocephalus is most often treated by surgically inserting a shunt system that diverts the flow of CSF from the ventricle to another area of the body where the CSF can be absorbed as part of the circulatory system. Shunt systems come in a variety of models, and typically share similar functional components. These components include a ventricular catheter which is introduced through a burr hole in the skull and implanted in the patient's ventricle, a drainage catheter that carries the CSF to its ultimate drainage site, and optionally a flow-control mechanism, e.g., shunt valve, that regulates the one-way flow of CSF from the ventricle to the drainage site to maintain normal pressure within the ventricles. The ventricular catheter typically contains multiple holes or pores positioned along the length of the ventricular catheter to allow the CSF to enter into the shunt system. To facilitate catheter insertion, a removable rigid stylet, situated within the lumen of the ventricular catheter, is used to direct the catheter toward the desired targeted location. Alternatively, or in addition, blunt tip brain cannulas and peel-away sheaths have been used to aid placement of the catheters.

One common problem encountered with the use of ventricular catheters is the difficulty in measuring the pressure within the patient's ventricle. One current technique involves placing a pressure sensor in line with the catheter and external to the ventricles such that the sensor communicates with the cerebrospinal fluid flowing through the catheter. As the pressure drop across the catheter is negligible, the sensor can measure pressure that resembles the intra-ventricular pressure. While this technique is advantageous in that it allows the use of a relatively large sensor, catheter blockage can impede the pressure sensed by the sensor, thus preventing an accurate measurement of intra-ventricular pressure from being obtained.

Accordingly, there remains a need for a catheter having a pressure sensor that is effective to accurately measure a patient's ventricular pressure.

EP-A-1 389 477 discloses a device for tip pressure monitoring for cryoablation catheters having some of the features of the present invention.

### SUMMARY OF THE INVENTION

The present invention generally provides pressure sensing ventricular catheters as defined in claims 1 to 5. The devices are particularly advantageous in that they provide a sensor assembly that is hermetically sealed, thereby allowing the device to be permanently implanted, and that can be effective to directly measure the intra- ventricular pressure, thus avoiding potentially inaccurate readings due to blockages occurring in the catheter.

The pressure sensor is coupled to the catheter using a variety of techniques. The pressure sensor can be disposed within a recess formed in an external surface of the elongate body. The pressure sensor can be embedded within the elongate body, and the elongate body can include an opening formed therein for exposing at least a portion of the pressure sensor to an external environment. The pressure sensor can be embedded within a distal tip of the elongate body, and a portion of the pressure sensor can protrude beyond the distal tip of the elongate body to measure a pressure of an external environment. The pressure sensor can be disposed within the inner lumen of the elongate body, and the elongate body can include an opening formed therein for exposing at least a portion of the pressure sensor to an external environment. The elongate body can include a second inner lumen extending therethrough, and the pressure sensor can be disposed within the second inner lumen of the elongate body and the elongate body includes an opening formed therein and extending into the second inner lumen for exposing at least a portion of the pressure sensor to an external environment.

In other aspects, at least a portion of the pressure sensing catheter can include a biocompatible fluid-impermeable coating for hermetically sealing the pressure sensor, the antenna, and/or a connector extending between the pressure sensor and the antenna. In one exemplary embodiment, the pressure sensor, antenna, and the connector extending therebetween can be coated to form a hermetically sealed sensor assembly. In other embodiments, only a portion of the pressure sensor, the antenna, and/or the connector can be coated. For example, a portion of the pressure sensor that is configured to be exposed to fluid during use can be coated. In yet another embodiment, the catheter can be coated such that any portion of the pressure sensor, the antenna, and the connector exposed to an external environment are coated. Where the catheter is coated, the fluid-entry ports formed in the catheter are preferably clear from any coating that would otherwise prevent fluid flow therethrough. The particular coating used can also vary, but in one exemplary embodiment the coating is a solvent-based silicone.

Methods for manufacturing intra-ventricular pressure sensor devices are also disclosed. In one exemplary embodiment, a catheter is formed having a distal end with a pressure sensor. At least a portion of the pressure sensor can be exposed to an ex- ternal environment such that the pressure sensor is adapted to measure a pressure surrounding the catheter. The catheter can also include an inner lumen extending therethrough, at least one fluid-entry port formed therein and in fluid communication with the inner lumen, an antenna coupled to the pressure sensor and adapted to communicate a measured pressure to an external device, and a connector extending between the pressure sensor and the antenna.

Where the antenna is embedded within the catheter, the catheter can be extruded with the antenna and at least a portion of the connector disposed therein. In other embodiments, the connector can be disposed within a slot formed in a sidewall of the catheter and extending along at least a portion of the length thereof. The method of manufacturing the catheter can also vary depending on the configuration of the pressure sensor. In one exemplary embodiment, the catheter can include a bullet-shaped tip that is coupled to a distal end thereof, and at least a portion of the pressure sensor can be disposed within the bullet- shaped tip. In another exemplary embodiment, the catheter can be manufactured by coupling the antenna, connector, and pressure sensor to form a sensor assembly, coating the sensor assembly such that it is fluid-impermeable, and coupling the sensor assembly to the catheter. The sensor assembly can be coupled to the catheter by, for example, attaching the sensor assembly to an external surface of the catheter, or positioning some of all of the sensor assembly in cutouts, slots, or recesses formed in the catheter.

### BRIEF DESCRIPTION OF THE DRAWINGS

The invention will be more fully understood from the following detailed description taken in conjunction with the accompanying drawings, in which:
FIG. 1 is a perspective view of one exemplary embodiment of a pressure sensing catheter having an external antenna not falling within the scope of the claims;
FIG. 2 is a cross-sectional view of an exemplary embodiment of a pressure sensing catheter having an internal antenna;
FIG. 3 is a cross-sectional view of one embodiment not according to the present invention of a distal portion of a pressure sensing catheter having a pressure sensor disposed on an external surface thereof;
FIG. 4 is a cross-sectional view of another embodiment of a distal portion of a pressure sensing catheter having a pressure sensor disposed within a recess formed therein;
FIG. 5 is a cross-sectional view of yet another embodiment of a distal portion of a pressure sensing catheter having a pressure sensor disposed within an inner lumen formed therein, and having a window formed therein for exposing a portion of the pressure sensor to an external environment;
FIG. 6 is a cross-sectional view of another embodiment of a distal portion of a pressure sensing catheter having a pressure sensor disposed within a second lumen formed in the catheter;
FIG. 7A is a cross-sectional view of yet another embodiment of a distal portion of a pressure sensing catheter having a pressure sensor disposed within a distal tip of the catheter;
FIG. 7B is a cross-sectional view of the distal tip of the catheter show in FIG. 7A taken across line B-B;
FIG. 8 is a top view of an exemplary embodiment of a pressure sensor;
FIG. 9A is a perspective view of an exemplary embodiment of an external radiofrequency telemetry system;
FIG. 9B is a perspective view of the external radiofrequency telemetry system of FIG. 9A in a disassembled configuration;
FIG. 10 is a cross-sectional view of a human brain showing one embodiment of a ventricular catheter not falling within the scope of the claims having a distal portion implanted in the ventricle and having an external antenna positioned under the patient's scalp;
FIG. 11 is a cross-sectional view of a human brain showing one embodiment of a ventricular catheter having a distal portion implanted in the ventricle and having an internal antenna positioned under the patient's scalp;

### DETAILED DESCRIPTION OF THE INVENTION

The present invention generally provides devices for measuring an intra-ventricular pressure. The devices are particularly advantageous in that they provide a hermetically sealed pressure sensing assembly that allows the device to be effective for long term or permanent implantation. In certain exemplary embodiment, the devices are also particularly advantageous in that they can be effective to obtain a direct measurement of the intra-ventricular pressure, thereby avoiding potentially inaccurate readings due to a blockage occurring in the ventricular catheter. A person skilled in the art will appreciate that, while the device is described in connection with a ventricular catheter for measuring the intra-ventricular pressure, the device can be used for a variety of medical procedures to measure the pressure in a variety of cavities.

In one exemplary embodiment, the present invention provides a pressure sensing catheter having a pressure sensor and an antenna that is coupled to the pressure sensor, e.g., by a connector. The pressure sensor can be adapted to measure a pressure surrounding the catheter, and the antenna can be adapted to telemetrically communicate the measured pressure to an external device. In an exemplary embodiment, the antenna, pressure sensor, and/or connector are hermetically sealed by the catheter and/or a coating to prevent the antenna, pressure sensor, and connector from coming into contact with fluid, thereby allowing the catheter to be permanently implanted or otherwise used for long term use. The catheter and/or coating are also particularly effective to prevent damage to the components due to the corrosive in-vivo environment. The catheter and/or coating should not, however, interfere with the ability of the pressure sensor to sense the pressure of fluid in contact therewith. Exemplary catheter configurations are discussed in the various embodiments described and illustrated herein, however a person having ordinary skill in the art will appreciate that the pressure sensing catheter can have a variety of other configurations. Moreover, the pressure sensor, antenna, and connector can be incorporated into virtually any catheter or other device.

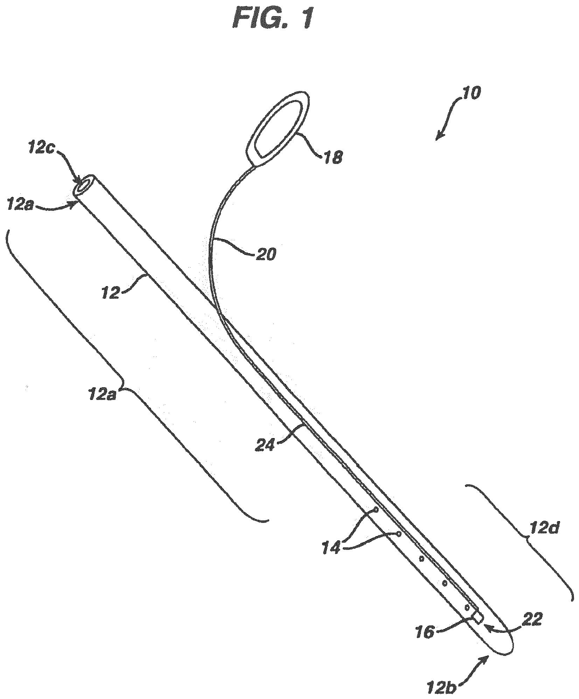

FIG. 1 illustrates one exemplary embodiment of a pressure sensing catheter 10 not falling within the scope of the claims. As shown, the catheter 10 has a generally elongate body 12 with proximal and distal ends 12a, 12b and an inner lumen 12c extending therethrough for accommodating fluid flow. The inner lumen 12c preferably terminates just proximal to the distal end 12b such that the distal end 12b is closed. The proximal end 12a can be open and it can be adapted to couple to another medical device, such as a valve for controlling fluid flow from the catheter 10. The catheter 10 can also include at least one fluid-entry port 14 formed in a sidewall thereof and extending into the inner lumen 12c. The location, quantity, and size of the fluid-entry ports 14 can vary, but they should be adapted to allow fluid to flow therethrough and into the inner lumen 12c.

The elongate body 12 that forms the catheter 10 can have virtually any configuration, shape, and size. Preferably, the elongate body 12 has a length I that is sufficient to allow at least the distal portion 12d to be implanted in a patient's ventricles, while the proximal portion 12p can extend therefrom to connect to another medical device, such as a valve. The elongate body 12 can also be formed from a variety of materials. In an exemplary embodiment, however, the body 12 is formed from a flexible, biocompatible material. Suitable materials include, for example, polymers such as silicones, polyethylene, and polyurethanes, all of which are known in the art. The body 12 can also optionally be formed from a radio-opaque material. A person skilled in the art will appreciate that the materials are not limited to those listed herein and that a variety of other biocompatible materials having the appropriate physical properties to enable the desired performance characteristics can be used.

As is further shown in FIG. 1, the catheter 10 also includes a pressure sensor 16 that is adapted to measure a pressure of an external environment, e.g., the pressure in the ventricle, surrounding the catheter 10, and an antenna 18 that is adapted to communicate the measured pressure to an external device. The pressure sensor 16 and the antenna 18 can be coupled to one another using a variety of techniques, but in the embodiment shown in FIG. 1 a connector 20 extends between the pressure sensor 16 and the antenna 18.

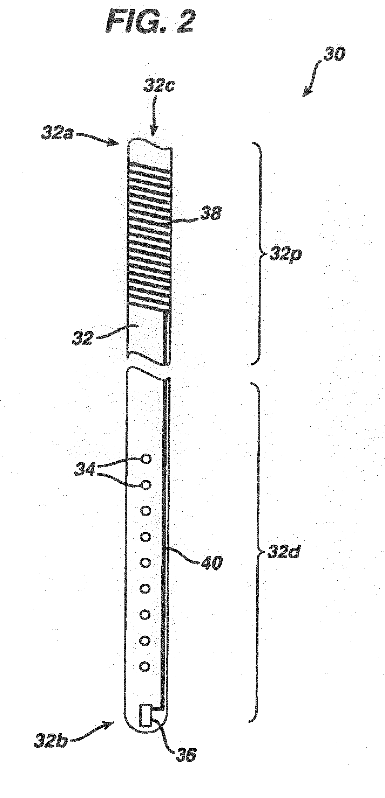

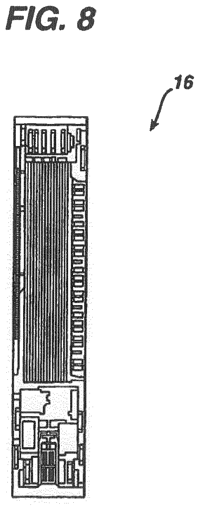

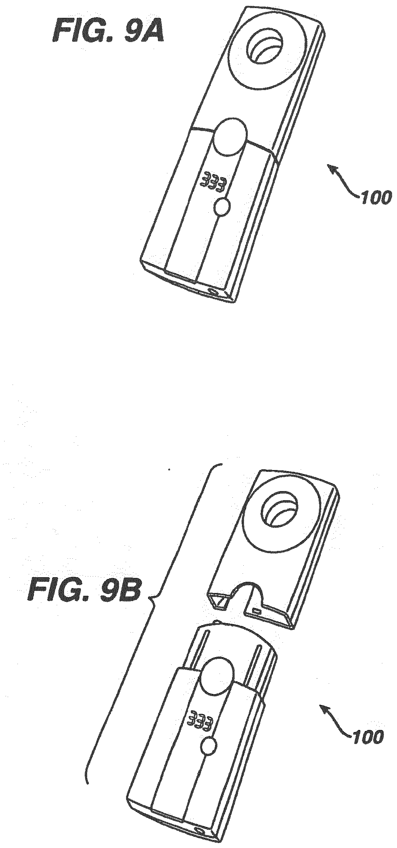

Exemplary embodiments of a pressure sensor 16, connector 20, and antenna 18 are described in more detail in U.S. Patent No. 5,321,989, U.S. Patent No. 5,431,057, and EP Patent No. 1 312 302. In general, the pressure sensor 16 can be formed on a microchip, as shown in FIG. 8. The size of the pressure sensor 16 can vary, but in one exemplary embodiment the pressure sensor 16 has a length of about 7 mm and a width of about 1 mm. The connector 20 that mates the pressure sensor 16 to the antenna 18 can also vary, but in an exemplary embodiment the connector 20 is a gold wire or other conductive element. The antenna 18 can also have a variety of configurations, but in an exemplary embodiment the antenna 18 preferably has a coil-shaped configuration. In particular, as shown in FIG. 1, the antenna 18 is formed into a substantially circular coil. Another embodiment of an antenna 38 is shown in FIG 2, and in that embodiment the antenna 38 is wrapped around the elongate body 12 to form a substantially cylindrical coil. The coil configuration will allow the antenna 18, 38 to function with an external device, such as the radio-frequency telemetry device 100 shown in FIG. 9A and 9B. The telemetry device is described in more detail in EP Patent No. 1 312 302.

Continuing to refer to FIG. 1, the particular configuration of the pressure sensor 16, connector 20, and antenna 18 with respect to the catheter 10 can vary. In the embodiment shown in FIG. 1, the pressure sensor 16 is disposed within a cut-out or recess 22 formed in the elongate body 12 adjacent to the distal end 12b. The recess 22 can extend partially through the elongate body 12, or it can extend fully through the elongate body 12 such that it is in communication with the inner lumen 12c. The position of the recess 22 can vary, but in an exemplary embodiment the recess 22 is formed distal of the distal-most fluid entry port 14. Such a configuration allows the pressure sensor 16 to be fully positioned within the ventricle to obtain an accurate reading.

As further shown in FIG. 1, the connector 20 is disposed within an elongate slot 24 formed in the elongate body 12 and extending proximally from the recess 22. A proximal portion of the connector 20 extends from the elongate slot 24 to mate to the antenna 18. As shown, the antenna 18 is not attached to the elongate body 12, but rather it is separate from the elongate body 12 and it is coupled to a proximal end of the connector 20. Such a configuration allows the antenna 18 to be positioned a distance apart from the elongate body 12. For example, when the pressure sensing catheter 10 is implanted in a patient's ventricle, the antenna 18 can be positioned just underneath the patient's scalp. This will allow an external device to be placed adjacent to the antenna 18 to telemetrically communicate with the antenna 18 and receive a pressure reading obtained by the pressure sensor 16, as will be discussed in more detail below. The elongate slot 24 also allows the connector 20 and antenna 18 to optionally be separated from the elongate body 12 if necessary. For example, the antenna 18 may need to be positioned a particular distance away from the elongate body 12, or the elongate body 12 may need to be trimmed, in which case an additional portion of the connector 20 can be removed from the slot 24.

As indicated above, the pressure sensor 16, connector 20, antenna 18, and/or the elongate body 12 can also include a coating that is adapted to hermetically seal all or at least a portion of the pressure sensor 16, connector 20, and antenna 18. The coating can be applied to only a portion of the pressure sensor 16, connector 20, and antenna 18 that will be exposed to fluid within the patient's ventricle, or it can be applied to each of the pressure sensor 16, connector 20, antenna 18, and optionally the elongate body 12. In the embodiment shown in FIG. 1, the pressure sensor 16, connector 20, and antenna 18, hereinafter collectively referred to as the sensor assembly, are preferably pre-coated prior to coupling the sensor assembly to the elongate body 12. Once coated the pressure sensor 16 can be positioned within the recess 22 and the connector 20 can be positioned within the slot 24. An adhesive or other mating technique can optionally be used to affix the pressure sensor 16 within the recess 22, and optionally to affix the connector 20 within the slot 24. Any adhesive or other mating technique can be used to attach the connector 20 to the elongate body 12, however, if desirable it should be configured to allow the connector 20 to be torn away from the body 12 if necessary.

Alternatively, or in addition to pre-coating, the catheter 10 can be coated after the sensor assembly is coupled to the elongate body 12 to form a protective sheath over the sensor assembly. The fluid-entry ports 14 should, however, either be protected from any coating applied thereto, formed after the coating is applied, or be cleared of any coating applied thereto to allow fluid to flow therethrough and into the inner lumen 12c. In other embodiments, only certain components of the sensor assembly can be coated. A person skilled in the art will appreciate that a variety of other techniques can be used to hermitically seal the pressure sensor 16, connector 20, and antenna 18.

The material used to form the coating can vary, and a variety of techniques can be used to apply the coating. By way of non-limiting example, suitable materials include polyurethane, silicone, solvent-based polymer solutions, and any other polymer that will adhere to the components to which it is applied to, and suitable techniques for applying the coating include spray-coating or dip-coating.

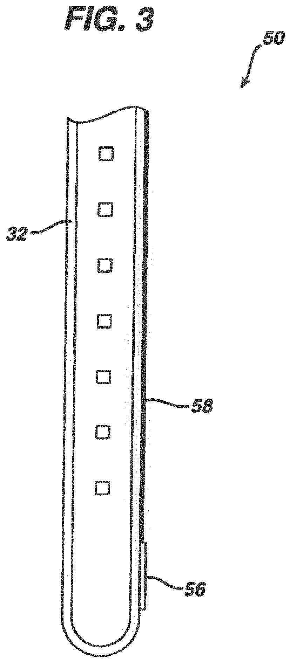

FIG. 2 illustrates an exemplary embodiment of a pressure sensing catheter 30 according to the invention. The catheter 30 is similar to catheter 10 in that it includes an elongate body 32 having proximal and distal ends 32a, 32b with an inner lumen 32c extending therebetween, and several fluid entry ports 34 formed in a distal portion 32d of the elongate body 32 and in fluid communication with the inner lumen 32c to allow fluid to flow therethrough. The catheter 30 also includes a pressure sensor 36 that is disposed within the distal end 32b of the elongate body 32 and a connector 40 that extends from the pressure sensor 36 to mate to an antenna 38. The pressure sensor 36 and connector 40 can be coupled to the elongate body 32 as previously described, or they can have a variety of other configurations as will be described in more detail with respect to FIGS. 3-7B.

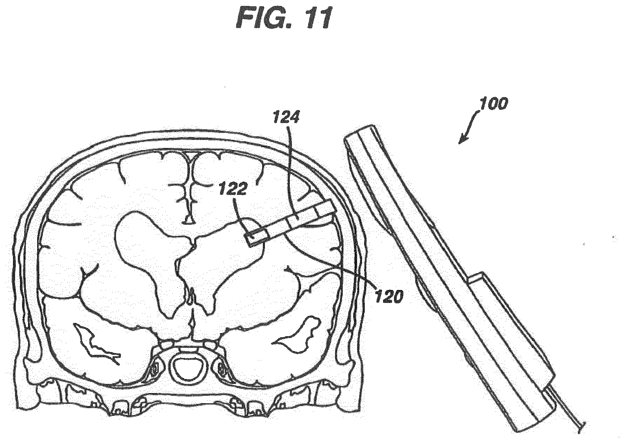

The embodiment shown in FIG. 2 differs from the embodiment shown in FIG. 1 in that the antenna 38 is not separate from and external to the elongate body 32, but rather it is coupled to a proximal portion 32p of the elongate body 32. In particular, the antenna 38 is formed into a cylindrical-shaped coil and is embedded within the elongate body 32. This can be achieved by molding the elongate body 32 around the antenna 38. Preferably, the connector 40 is coupled to the antenna 38 and is also molded within the elongate body 32. In other embodiments, the antenna 38 can be wrapped around the elongate body 32 or it can be disposed within the inner lumen 32c of the elongate body 32. An adhesive can optionally be used to fix the antenna 18 to the elongate body 12. In use, as will be discussed in more detail with respect to FIG. 11, the antenna 38, which is disposed in the proximal portion 32p of the elongate body 32, can be positioned outside of the ventricle and adjacent to the patient's scalp to allow a reading to be obtained from the pressure sensor 32 via the antenna 38. Accordingly, in an exemplary embodiment, the antenna 38 should be positioned at a location on the elongate body 32 that allows for such positioning of the antenna 38 during use.

As previously discussed with respect to FIG. 1, the catheter 30 can also include a coating applied to the pressure sensor 36, antenna 38, connector 40, and/or the elongate body 32. In an exemplary embodiment, the coating is only applied to the pressure sensor 36, or at least to the portion of the pressure sensor 36 that is exposed to the external environment, as the catheter forms a seal around the connector 40 and antenna 38 that are embedded therein. For example, the pressure sensor 36 can be disposed within a recess formed in the elongate body 32 and the coating can be applied over the recess to seal the pressure sensor 36 therein.

FIGS. 3-7B illustrate a variety of other configurations for the pressure sensor and connector with respect to the elongate body. While not shown, the antenna coupling to the pressure sensor and connector can be coupled to the catheter, or it can be separate from and external to the catheter. While the pressure sensor and connector can have a variety of configurations, in certain exemplary embodiments the pressure sensor and/or connector can be positioned within the inner lumen of the elongate body, positioned within a second inner lumen in the elongate body, embedded in the elongate body, disposed on an external surface of the elongate body, and/or positioned external to the elongate body. A coating can be applied to the pressure sensor and/or connector, however the particular technique used to hermetically seal the pressure sensor and connector in each embodiment can depend on the configuration of the pressure sensor and connector, as well as the antenna (shown in FIGS. 1 and 2), and whether the antenna is internal or external to the elongate body. Exemplary methods for manufacturing each embodiment and for hermetically sealing the pressure sensor and connector are also disclosed. A person skilled in the art will appreciate that the pressure sensing catheter can have a variety of configurations, and that the method for manufacturing the pressure sensing catheter can vary depending on the configuration.

FIG. 3 illustrates a distal portion 50 of an elongate body 52 of a pressure sensing catheter. In this embodiment, which is not according to the present invention, the pressure sensor 56 and the connector 58 are disposed on an external surface of the elongate body 52. An adhesive or any other mating technique can be used to attach the pressure sensor 56 and connector 58 to the body 52. In order to protect the sensor 56 and connector 58 from fluid damage, a coating can be applied to some or all of the device, as previously described. For example, the sensor assembly can be pre-coated prior to coupling the sensor assembly to the elongate body 52. Alternatively, or in addition, a coating can be applied to the elongate body 52 after the sensor assembly is coupled thereto. The fluid-entry ports should, of course, be free from any coating to allowing fluid to flow therethrough.

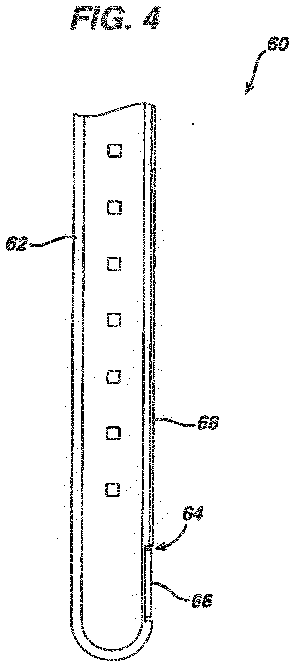

FIG. 4 illustrates an embodiment according to the invention of a distal portion 60 of an elongate body 62 of a pressure sensing catheter. In this embodiment, which is similar to the embodiment shown in FIG. 1, the pressure sensor 66 is disposed within a recess 64 formed in the elongate body 62, and the connector 68 is embedded within the elongate body 62. The connector 68 can be embedded in the elongate body 62 during molding, and the recess 64 can either be molded into the elongate body 62 or it can be cut out of the elongate body 62 after the elongate body 62 is formed. The sensor 66 can thereafter be positioned within the recess 64. As previously discussed, an adhesive or any other mating technique can be used to retain the pressure sensor 66 within the recess 64. A coating can be applied to the pressure sensor 66 prior to and/or after disposing the pressure sensor 66 in the recess 64. The coating can optionally be applied to the elongate body 62 to seal the pressure sensor 66 within the recess 64.

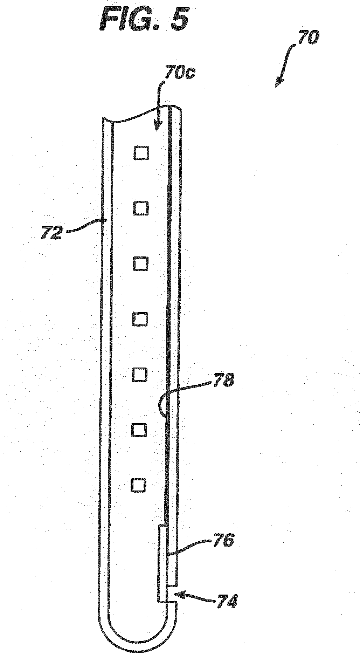

FIG. 5 illustrates another embodiment according to the invention, of a distal portion 70 of an elongate body 72 of a pressure sensing catheter. In this embodiment, the pressure sensor 76 and the connector 78 are disposed within the inner lumen 70c of the elongate body 72. A cut-out or window 74 is formed in the elongate body 72 to expose a portion of the pressure sensor 76 that is adapted to measure the pressure surrounding the catheter. A coating is preferably disposed over the sensor assembly before it is disposed within the elongate body 72, and an adhesive of other mating technique can be used to attach the sensor assembly to the elongate body 72. A coating can optionally be applied to the window 74.

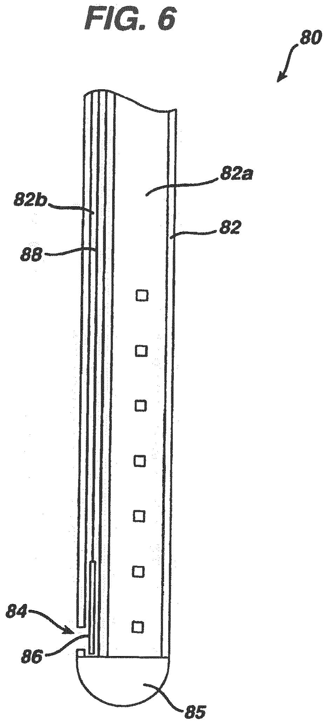

FIG. 6 illustrates another embodiment according to the invention of a distal portion 80 of an elongate body 82 of a pressure sensing catheter. In this embodiment, the elongate body 82 includes first and second inner lumens 82a, 82b, and the pressure sensor 86 and the connector 88 are disposed within the second inner lumen 82b. A cut-out or window 84 is formed in the elongate body 82 and is in commu- nication with the second inner lumen 82b to expose a portion of the pressure sensor 86 that is adapted to meas- ure the pressure surrounding the catheter. A coating can either be disposed over the sensor assembly before it is disposed within the second inner lumen 82b in the elongate body 82, or a coating can be applied to the window 84 to seal the pressure sensor 86 and connector 88 within the elongate body 82.

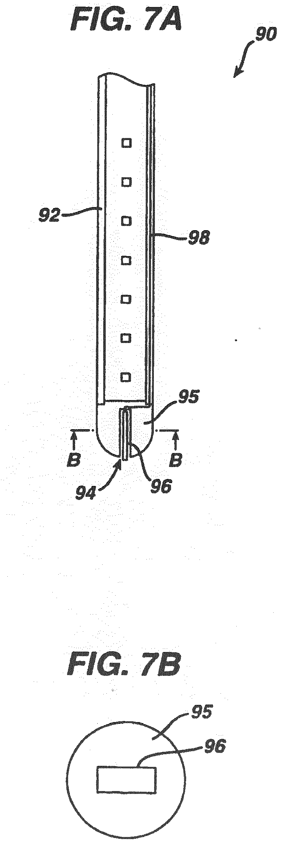

FIGS. 7A and 7B illustrate another embodiment according to the invention of a distal portion 90 of an elongate body 92 of a pressure sensing catheter. In this embodiment, the pressure sensor 96 is disposed within a bullet-shaped tip 95 that is attached to the elongate body 92. The bullet-shaped tip 95 and elongate body 92 can be manufactured as separate components and then coupled to one another to couple the pressure sensor 96 to the connector 98. As shown in FIGS. 7A and 7B, the pressure sensor 96 is disposed within a recess 94 formed in the distal-most end of the bullet-shaped tip 95, however a portion of the pressure sensor 96 preferably extends from the recess 94 to allow the external pressure to be measured. An adhesive can be used to retain the pressure sensor 96 within the tip 95, and a coating can be applied to the pressure sensor 96 and/or the tip 95. As is further shown in FIG. 7A, the elongate body 92 can be formed with the connector 98 embedded therein. An adhesive or other mating technique can then be used to attach the bullet- shaped tip 95 to the open distal end of the elongate body 92. The connector 98 disposed within the elongate body 92 can then be coupled to the pressure sensor 96 in the tip 95. Various techniques can be used to achieve a connection. For example, a portion of the connector 98 can be embedded or disposed within the tip 95 and connected to the pressure sensor 96 when the pressure sensor 96 is inserted into the recess 94. The two portions of the connector 98 can then be mated to one another when the tip 95 is attached to the elongate body 92. Alternatively, the recess can extend completely through the distal tip 92 to allow a portion of the connector 98 extending from the elongate body 92 to be connected to the pressure sensor 96. As previously discussed, various techniques can be used to hermetically seal the pressure sensor 96, connector 98, and antenna (shown in FIGS. 1 and 2).

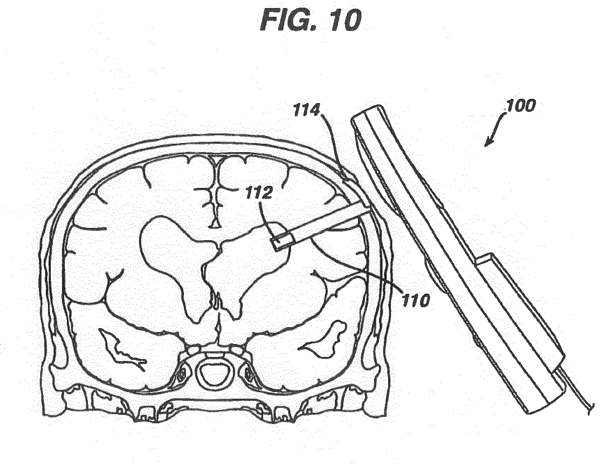

FIGS. 10 and 11 shown exemplary methods for using a pressure sensing catheter. Referring first to FIG. 10, a pressure sensing catheter 110 not falling within the scope of the claims is shown having a pressure sensor 112 (labeled "chip") disposed within the distal end thereof, and an external antenna 114 (labeled "coil") positioned a distance apart from a proximal end thereof. As shown, the catheter 110 is positioned in the patient's ventricle, and the external antenna 114 is positioned just beneath just beneath the patient's scalp. The pressure sensor 112, which is exposed to the fluid surrounding the catheter 110, can measure the ventricular pressure surrounding the catheter 110. An external device 100, as shown, can then be positioned adjacent to the antenna 114 to telemetrically communicate with the antenna 114, and thereby obtain a reading of the measured pressure.

FIG. 11 similarly illustrates a pressure sensing catheter 120 having a pressure sensor 112 disposed within a patient's ventricle for measuring the intra-ventricular pressure. In this embodiment, the antenna 124 is internal to the catheter 120, however it is still positioned just beneath the patient's scalp. The device 120 therefore functions in the same manner described above with respect to FIG. 10.

Multiple pressure sensor assemblies can also be used, and they can be disposed
at various locations relative to one another. The use of multiple pressure sensor assemblies can be particularly advantageous as it can allow a differential pressure of the system to be obtained. The differential pressure of the system should be equal to the operating pressure of the system, thus indicating whether the system is performing properly.

## Claims

1. A pressure sensing ventricular catheter (10) for measuring intra-ventricular pressure in a brain comprising:
an elongate body (12) having an inner lumen (12c) extending at least partially therethrough, a distal portion having a distal end having a pressure sensor (16), at least a portion of the pressure sensor (16) being exposed to an external environment surrounding the catheter (10) such that the pressure sensor (16) is effective to measure the pressure of the external environment, wherein the pressure sensor (16) is disposed within a recess formed in an external surface of the elongate body (12);
at least one fluid-entry port (14, 34) formed in the elongate body proximal to the pressure sensor and in fluid communication with the inner lumen (12c); and
an antenna (18) coupled to the pressure sensor (16) and adapted to communicate a measured pressure from the pressure sensor (16) to an external device;
wherein the antenna (18) comprises a coil coupled to the pressure sensor (16) by a connector, and the coil is either:
(a) disposed around the elongate body (12); or
(b) embedded within the elongate body (12).

2. A pressure sensing ventricular catheter (10) for measuring intra-ventricular pressure in a brain comprising:
an elongate body (12) having an inner lumen (12c) extending at least partially therethrough, a distal portion having a distal end having a pressure sensor (16), at least a portion of the pressure sensor (16) being exposed to an external environment surrounding the catheter (10) such that the pressure sensor (16) is effective to measure the pressure of the external environment, wherein the pressure sensor (16) is embedded within the elongate body (12), and wherein the elongate body (12) includes an opening formed therein for exposing at least a portion of the pressure sensor (16) to an external environment;
at least one fluid-entry port (14, 34) formed in the elongate body proximal to the pressure sensor and in fluid communication with the inner lumen (12c); and
an antenna (18) coupled to the pressure sensor (16) and adapted to communicate a measured pressure from the pressure sensor (16) to an external device;
wherein the antenna (18) comprises a coil coupled to the pressure sensor (16) by a connector, and the coil is either:
(a) disposed around the elongate body (12); or
(b) embedded within the elongate body (12).

3. A pressure sensing ventricular catheter (10) for measuring intra-ventricular pressure in a brain comprising:
an elongate body (12) having an inner lumen (12c) extending at least partially therethrough, a distal portion having a distal end having a pressure sensor (16), at least a portion of the pressure sensor (16) being exposed to an external environment surrounding the catheter (10) such that the pressure sensor (16) is effective to measure the pressure of the external environment, wherein the pressure sensor (16) is embedded within a distal tip of the elongate body (12), and wherein the portion of the pressure sensor (16) protrudes beyond the distal tip of the elongate body (12) to measure a pressure of an external environment;
at least one fluid-entry port (14, 34) formed in the elongate body proximal to the pressure sensor and in fluid communication with the inner lumen (12c); and
an antenna (18) coupled to the pressure sensor (16) and adapted to communicate a measured pressure from the pressure sensor (16) to an external device;
wherein the antenna (18) comprises a coil coupled to the pressure sensor (16) by a connector, and the coil is either:
(a) disposed around the elongate body (12); or
(b) embedded within the elongate body (12).

4. A pressure sensing ventricular catheter (10) for measuring intra-ventricular pressure in a brain comprising:
an elongate body (12) having an inner lumen (12c) extending at least partially therethrough, a distal portion having a distal end having a pressure sensor (16), at least a portion of the pressure sensor (16) being exposed to an external environment surrounding the catheter (10) such that the pressure sensor (16) is effective to measure the pressure of the external environment, wherein the pressure sensor (16) is disposed within the inner lumen (12c) of the elongate body (12), and wherein the elongate body (12) includes an opening formed therein for exposing at least a portion of the pressure sensor (16) to an external environment;
at least one fluid-entry port (14, 34) formed in the elongate body proximal to the pressure sensor and in fluid communication with the inner lumen (12c); and
an antenna (18) coupled to the pressure sensor (16) and adapted to communicate a measured pressure from the pressure sensor (16) to an external device;
wherein the antenna (18) comprises a coil coupled to the pressure sensor (16) by a connector, and the coil is either:
(a) disposed around the elongate body (12); or
(b) embedded within the elongate body (12).

5. A pressure sensing ventricular catheter (10) for measuring intra-ventricular pressure in a brain comprising:
an elongate body (12) having an inner lumen (12c) extending at least partially therethrough, a distal portion having a distal end having a pressure sensor (16), at least a portion of the pressure sensor (16) being exposed to an external environment surrounding the catheter (10) such that the pressure sensor (16) is effective to measure the pressure of the external environment, wherein the elongate body (12) includes a second inner lumen extending therethrough, and wherein the pressure sensor (16) is disposed within the second inner lumen of the elongate body (12), the elongate body (12) including an opening formed therein and extending into the second inner lumen for exposing at least a portion of the pressure sensor (16) to an external environment;
at least one fluid-entry port (14, 34) formed in the elongate body proximal to the pressure sensor and in fluid communication with the inner lumen (12c); and
an antenna (18) coupled to the pressure sensor (16) and adapted to communicate a measured pressure from the pressure sensor (16) to an external device;
wherein the antenna (18) comprises a coil coupled to the pressure sensor (16) by a connector, and the coil is either:
(a) disposed around the elongate body (12); or
(b) embedded within the elongate body (12).

6. The device of any of claims 1 to 5, wherein at least the exposed portion of the pressure sensor (16) includes biocompatible fluid-impermeable coating disposed thereon.

7. The device of claim 6, wherein the biocompatible fluid-impermeable coating is disposed on the pressure sensor (16) and the antenna (18) such that the pressure sensor (16) and the antenna (18) are fluid impermeable.

## Patentansprüche

1. Druckabfühlender ventrikularer Katheter (10) zum Messen des intraventrikularen Drucks in einem Gehirn, umfassend:
einen länglichen Körper (12) mit einem sich mindestens teilweise dort hindurch erstreckenden inneren Lumen (12c), wobei ein distaler Abschnitt ein distales Ende mit einem Drucksensor (16) hat, wobei mindestens ein Abschnitt des Drucksensors (16) einer externen Umgebung ausgesetzt ist, die den Katheter (10) umgibt, so dass der Drucksensor (16) so wirkt, dass er den Druck der externen Umgebung misst, wobei der Drucksensor (16) in einer Aussparung angeordnet ist, die in einer Außenfläche des länglichen Körpers (12) ausgebildet ist,
mindestens einen Fluideintrittsport (14, 34), der in dem länglichen Körper proximal zu dem Drucksensor ausgebildet ist und in Fluidverbindung mit dem inneren Lumen (12c) steht, und
eine Antenne (18), die an den Drucksensor (16) gekoppelt und dazu ausgeführt ist, einen gemessenen Druck von dem Drucksensor (16) an eine externe Vorrichtung zu kommunizieren,
wobei die Antenne (18) eine Spule aufweist, die über einen Verbinder an den Drucksensor (16) gekoppelt ist, und die Spule entweder:
(a) um den länglichen Körper (12) angeordnet ist; oder
(b) in dem länglichen Körper (12) eingebettet ist.

2. Druckabfühlender ventrikularer Katheter (10) zum Messen des intraventrikularen Drucks in einem Gehirn, umfassend:
einen länglichen Körper (12) mit einem sich mindestens teilweise dort hindurch erstreckenden inneren Lumen (12c), wobei ein distaler Abschnitt ein distales Ende mit einem Drucksensor (16) hat, wobei mindestens ein Abschnitt des Drucksensors (16) einer externen Umgebung ausgesetzt ist, die den Katheter (10) umgibt, so dass der Drucksensor (16) so wirkt, dass er den Druck der externen Umgebung misst, wobei der Drucksensor (16) in dem länglichen Körper (12) eingebettet ist und wobei der längliche Körper (12) eine in ihm ausgebildete Öffnung zum Freilegen mindestens eines Abschnitts des Drucksensors (16) gegenüber einer externen Umgebung aufweist,
mindestens einen Fluideintrittsport (14, 34), der in dem länglichen Körper proximal zu dem Drucksensor ausgebildet ist und in Fluidverbindung mit dem inneren Lumen (12c) steht, und
eine Antenne (18), die an den Drucksensor (16) gekoppelt und dazu ausgeführt ist, einen gemessenen Druck von dem Drucksensor (16) an eine externe Vorrichtung zu kommunizieren,
wobei die Antenne (18) eine Spule aufweist, die über einen Verbinder an den Drucksensor (16) gekoppelt ist, und die Spule entweder:
(a) um den länglichen Körper (12) angeordnet ist; oder
(b) in dem länglichen Körper (12) eingebettet ist.

3. Druckabfühlender ventrikularer Katheter (10) zum Messen des intraventrikularen Drucks in einem Gehirn, umfassend:
einen länglichen Körper (12) mit einem sich mindestens teilweise dort hindurch erstreckenden inneren Lumen (12c), wobei ein distaler Abschnitt ein distales Ende mit einem Drucksensor (16) hat, wobei mindestens ein Abschnitt des Drucksensors (16) einer externen Umgebung ausgesetzt ist, die den Katheter (10) umgibt, so dass der Drucksensor (16) so wirkt, dass er den Druck der externen Umgebung misst, wobei der Drucksensor (16) in einer distalen Spitze des länglichen Körpers (12) eingebettet ist und wobei der Abschnitt des Drucksensors (16) über die distale Spitze des länglichen Körpers (12) hinaus vorragt, um einen Druck einer externen Umgebung zu messen,
mindestens einen Fluideintrittsport (14, 34), der in dem länglichen Körper proximal zu dem Drucksensor ausgebildet ist und in Fluidverbindung mit dem inneren Lumen (12c) steht, und
eine Antenne (18), die an den Drucksensor (16) gekoppelt und dazu ausgeführt ist, einen gemessenen Druck von dem Drucksensor (16) an eine externe Vorrichtung zu kommunizieren,
wobei die Antenne (18) eine Spule aufweist, die über einen Verbinder an den Drucksensor (16) gekoppelt ist, und die Spule entweder:
(a) um den länglichen Körper (12) angeordnet ist; oder
(b) in dem länglichen Körper (12) eingebettet ist.

4. Druckabfühlender ventrikularer Katheter (10) zum Messen des intraventrikularen Drucks in einem Gehirn, umfassend:
einen länglichen Körper (12) mit einem sich mindestens teilweise dort hindurch erstreckenden inneren Lumen (12c), wobei ein distaler Abschnitt ein distales Ende mit einem Drucksensor (16) hat, wobei mindestens ein Abschnitt des Drucksensors (16) einer externen Umgebung ausgesetzt ist, die den Katheter (10) umgibt, so dass der Drucksensor (16) so wirkt, dass er den Druck der externen Umgebung misst, wobei der Drucksensor (16) in dem inneren Lumen (12c) des länglichen Körpers (12) angeordnet ist und wobei der längliche Körper (12) eine in ihm ausgebildete Öffnung zum Freilegen mindestens eines Abschnitts des Drucksensors (16) gegenüber einer externen Umgebung aufweist,
mindestens einen Fluideintrittsport (14, 34), der in dem länglichen Körper proximal zu dem Drucksensor ausgebildet ist und in Fluidverbindung mit dem inneren Lumen (12c) steht, und
eine Antenne (18), die an den Drucksensor (16) gekoppelt und dazu ausgeführt ist, einen gemessenen Druck von dem Drucksensor (16) an eine externe Vorrichtung zu kommunizieren,
wobei die Antenne (18) eine Spule aufweist, die über einen Verbinder an den Drucksensor (16) gekoppelt ist, und die Spule entweder:
(a) um den länglichen Körper (12) angeordnet ist; oder
(b) in dem länglichen Körper (12) eingebettet ist.

5. Druckabfühlender ventrikularer Katheter (10) zum Messen des intraventrikularen Drucks in einem Gehirn, umfassend:
einen länglichen Körper (12) mit einem sich mindestens teilweise dort hindurch erstreckenden inneren Lumen (12c), wobei ein distaler Abschnitt ein distales Ende mit einem Drucksensor (16) hat, wobei mindestens ein Abschnitt des Drucksensors (16) einer externen Umgebung ausgesetzt ist, die den Katheter (10) umgibt, so dass der Drucksensor (16) so wirkt, dass er den Druck der externen Umgebung misst, wobei der längliche Körper (12) ein sich dort hindurch erstreckendes zweites inneres Lumen aufweist und wobei der Drucksensor (16) in dem zweiten inneren Lumen des länglichen Körpers (12) angeordnet ist, wobei der längliche Körper (12) eine in ihm ausgebildete und sich in das zweite innere Lumen erstreckende Öffnung zum Freilegen mindestens eines Abschnitts des Drucksensors (16) gegenüber einer externen Umgebung aufweist,
mindestens einen Fluideintrittsport (14, 34), der in dem länglichen Körper proximal zu dem Drucksensor ausgebildet ist und in Fluidverbindung mit dem inneren Lumen (12c) steht, und
eine Antenne (18), die an den Drucksensor (16) gekoppelt und dazu ausgeführt ist, einen gemessenen Druck von dem Drucksensor (16) an eine externe Vorrichtung zu kommunizieren,
wobei die Antenne (18) eine Spule aufweist, die über einen Verbinder an den Drucksensor (16) gekoppelt ist, und die Spule entweder:
(a) um den länglichen Körper (12) angeordnet ist; oder
(b) in dem länglichen Körper (12) eingebettet ist.

6. Vorrichtung nach Ansprüchen 1 bis 5, wobei mindestens der freiliegende Abschnitt des Drucksensors (16) einen biokompatiblen, fluidundurchlässigen Überzug aufweist, der auf ihm angeordnet ist.

7. Vorrichtung nach Anspruch 6, wobei der biokompatible, fluidundurchlässige Überzug auf dem Drucksensor (16) und der Antenne (18) derart angeordnet ist, dass der Drucksensor (16) und die Antenne (18) fluidundurchlässig sind.

## Revendications

1. Cathéter ventriculaire de détection de pression (10) pour mesurer la pression intra-ventriculaire dans un cerveau, comprenant :
un corps allongé (12) ayant une lumière interne (12c) s'étendant au moins partiellement à travers celui-ci, une partie distale ayant une extrémité distale ayant un capteur de pression (16), au moins une partie du capteur de pression (16) étant exposée à un environnement extérieur entourant le cathéter (10) de telle sorte que le capteur de pression (16) puisse mesurer effectivement la pression de l'environnement extérieur, le capteur de pression (16) étant disposé à l'intérieur d'un renfoncement formé dans une surface extérieure du corps allongé (12) ;
au moins un orifice d'entrée de fluide (14, 34) formé dans le corps allongé à proximité du capteur de pression et en communication de fluide avec la lumière interne (12c), et
une antenne (18) couplée au capteur de pression (16) et adaptée pour communiquer une pression mesurée provenant du capteur de pression (16) à un dispositif externe ;
l'antenne (18) comprenant une bobine couplée au capteur de pression (16) par un connecteur et la bobine étant soit :
(a) disposée autour du corps allongé (12) ; soit
(b) encastrée à l'intérieur du corps allongé (12) .

2. Cathéter ventriculaire de détection de pression (10) pour mesurer la pression intra-ventriculaire dans un cerveau, comprenant :
un corps allongé (12) ayant une lumière interne (12c) s'étendant au moins partiellement à travers celui-ci, une partie distale ayant une extrémité distale ayant un capteur de pression (16), au moins une partie du capteur de pression (16) étant exposée à un environnement extérieur entourant le cathéter (10) de telle sorte que le capteur de pression (16) puisse mesurer effectivement la pression de l'environnement extérieur, le capteur de pression (16) étant encastré à l'intérieur du corps allongé (12), et le corps allongé (12) comportant une ouverture formée dans celui-ci pour exposer au moins une partie du capteur de pression (16) à un environnement extérieur ;
au moins un orifice d'entrée de fluide (14, 34) formé dans le corps allongé à proximité du capteur de pression et en communication de fluide avec la lumière interne (12c), et
une antenne (18) couplée au capteur de pression (16) et adaptée pour communiquer une pression mesurée provenant du capteur de pression (16) à un dispositif externe ;
l'antenne (18) comprenant une bobine couplée au capteur de pression (16) par un connecteur et la bobine étant soit :
(a) disposée autour du corps allongé (12) ; soit
(b) encastrée à l'intérieur du corps allongé (12) .

3. Cathéter ventriculaire de détection de pression (10) pour mesurer la pression intra-ventriculaire dans un cerveau, comprenant :
un corps allongé (12) ayant une lumière interne (12c) s'étendant au moins partiellement à travers celui-ci, une partie distale ayant une extrémité distale ayant un capteur de pression (16), au moins une partie du capteur de pression (16) étant exposée à un environnement extérieur entourant le cathéter (10) de telle sorte que le capteur de pression (16) puisse mesurer effectivement la pression de l'environnement extérieur, le capteur de pression (16) étant encastré à l'intérieur d'une pointe distale du corps allongé (12), et la partie du capteur de pression (16) faisant saillie au-delà de la pointe distale du corps allongé (12) pour mesurer une pression d'un environnement extérieur ;
au moins un orifice d'entrée de fluide (14, 34) formé dans le corps allongé à proximité du capteur de pression et en communication de fluide avec la lumière interne (12c), et
une antenne (18) couplée au capteur de pression (16) et adaptée pour communiquer une pression mesurée provenant du capteur de pression (16) à un dispositif externe ;
l'antenne (18) comprenant une bobine couplée au capteur de pression (16) par un connecteur et la bobine étant soit :
(a) disposée autour du corps allongé (12) ; soit
(b) encastrée à l'intérieur du corps allongé (12) .

4. Cathéter ventriculaire de détection de pression (10) pour mesurer la pression intra-ventriculaire dans un cerveau, comprenant :
un corps allongé (12) ayant une lumière interne (12c) s'étendant au moins partiellement à travers celui-ci, une partie distale ayant une extrémité distale ayant un capteur de pression (16), au moins une partie du capteur de pression (16) étant exposée à un environnement extérieur entourant le cathéter (10) de telle sorte que le capteur de pression (16) puisse mesurer effectivement la pression de l'environnement extérieur, le capteur de pression (16) étant disposé à l'intérieur de la lumière interne (12c) du corps allongé (12), et le corps allongé (12) comportant une ouverture formée dans celui-ci pour exposer au moins une partie du capteur de pression (16) à un environnement extérieur ;
au moins un orifice d'entrée de fluide (14, 34) formé dans le corps allongé à proximité du capteur de pression et en communication de fluide avec la lumière interne (12c), et
une antenne (18) couplée au capteur de pression (16) et adaptée pour communiquer une pression mesurée provenant du capteur de pression (16) à un dispositif externe ;
l'antenne (18) comprenant une bobine couplée au capteur de pression (16) par un connecteur et la bobine étant soit :
(a) disposée autour du corps allongé (12) ; soit
(b) encastrée à l'intérieur du corps allongé (12) .

5. Cathéter ventriculaire de détection de pression (10) pour mesurer la pression intra-ventriculaire dans un cerveau, comprenant :
un corps allongé (12) ayant une lumière interne (12c) s'étendant au moins partiellement à travers celui-ci, une partie distale ayant une extrémité distale ayant un capteur de pression (16), au moins une partie du capteur de pression (16) étant exposée à un environnement extérieur entourant le cathéter (10) de telle sorte que le capteur de pression (16) puisse mesurer effectivement la pression de l'environnement extérieur, le corps allongé (12) comportant une deuxième lumière interne s'étendant à travers lui, et le capteur de pression (16) étant disposé à l'intérieur de la deuxième lumière interne du corps allongé (12), le corps allongé (12) comportant une ouverture formée dans celui-ci et s'étendant dans la deuxième lumière interne pour exposer au moins une partie du capteur de pression (16) à un environnement extérieur ;
au moins un orifice d'entrée de fluide (14, 34) formé dans le corps allongé à proximité du capteur de pression et en communication de fluide avec la lumière interne (12c), et
une antenne (18) couplée au capteur de pression (16) et adaptée pour communiquer une pression mesurée provenant du capteur de pression (16) à un dispositif externe ;
l'antenne (18) comprenant une bobine couplée au capteur de pression (16) par un connecteur et la bobine étant soit :
(a) disposée autour du corps allongé (12) ; soit
(b) encastrée à l'intérieur du corps allongé (12) .

6. Dispositif selon l'une quelconque des revendications 1 à 5, dans lequel au moins la partie exposée du capteur de pression (16) comprend un revêtement imperméable aux fluides biocompatible disposé sur celui-ci.

7. Dispositif selon la revendication 6, dans lequel le revêtement imperméable aux fluides biocompatible est disposé sur le capteur de pression (16) et l'antenne (18) de sorte que le capteur de pression (16) et l'antenne (18) soient imperméables aux fluides.
